# EUROPEAN PATENT APPLICATION

(11) **EP 1 685 814 A1**
(43) Date of publication of application: **02.08.2006**
(21) Application number: 05250527.8
(22) Date of filing: 01.02.2005
(51) Int. Cl.: A61F 5/01, A62B 1/10, B66D 5/02

(54) **Pressure activated ratchet**

(71) Applicant: Fikes, Ray, Fountain Hills, Arizona 85261 (US)
(72) Inventor: Fikes, Ray, Fountain Hills, Arizona 85261 (US)
(74) Representative: Johnstone, Helen Margaret

(57) **Abstract**

A mechanism including a ratchet member (12) mounted for movement in two opposite directions and a second member (30) mounted adjacent the ratchet member, the second member responsive to predetermined pressure (via 34) to engage the ratchet member (12) to constrain movement thereof in one of the two opposite directions.

## Description

This invention relates to a ratchet mechanism.
Figure 1 is an exploded view of a ratchet mechanism according to an embodiment of the present invention;
Figure 2 is a top view of various components of the embodiment shown in Figure 1;
Figures 3 and 4 are top views of portions of the embodiment shown in Figures 1 and 2, illustrating features of operation thereof;
Figure 5 is a top view of the assembled embodiment shown in Figure 1;
Figure 6 is a perspective view of an orthopedic back brace with which the embodiment of Figures 1 through 5 may be used;
Figure 7 illustrates an operating position of the back brace shown in Figure 6; and
Figure 8 illustrates a different operating position of the back brace of Figures 6 and 7 useful in conjunction with the description of the embodiment of Figures 1 through 5.

### DETAILED DESCRIPTION:

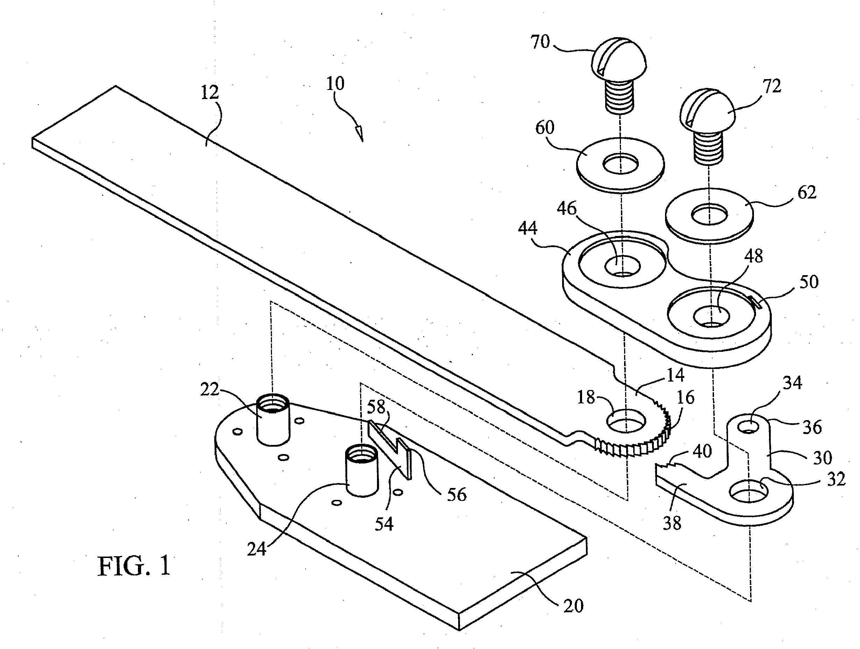

Reference now should be made to the drawings, in which the same reference numbers are used throughout the different figures to designate the same or similar components. Figure 1 is an exploded view of a pressure activated ratchet mechanism which may be incorporated into a variety of different products to provide a useful motion restriction mechanism. Some examples of uses which may incorporate the embodiment shown in Figures 1 through 5 are spinal hypertension braces, ratcheting joints in cervical braces, lounge and pool chairs which require various relative positions between the seat and the back, ratcheting joints in knee braces and prostheses, winches, such as used in boat launch mechanisms, ratcheting joints in ankle-foot braces and prostheses, ratcheting joints in upper extremity braces and prostheses, camping gear, and other mechanisms which require positioning and secure fastening.

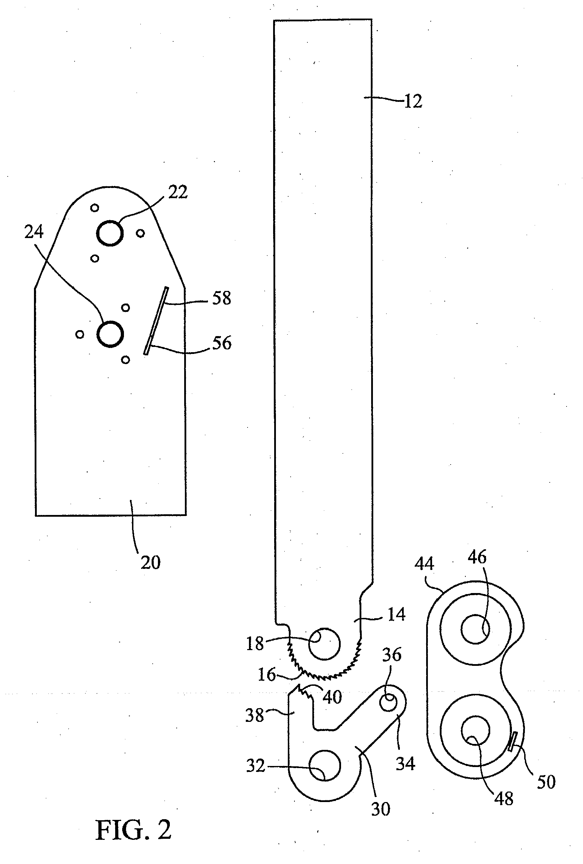

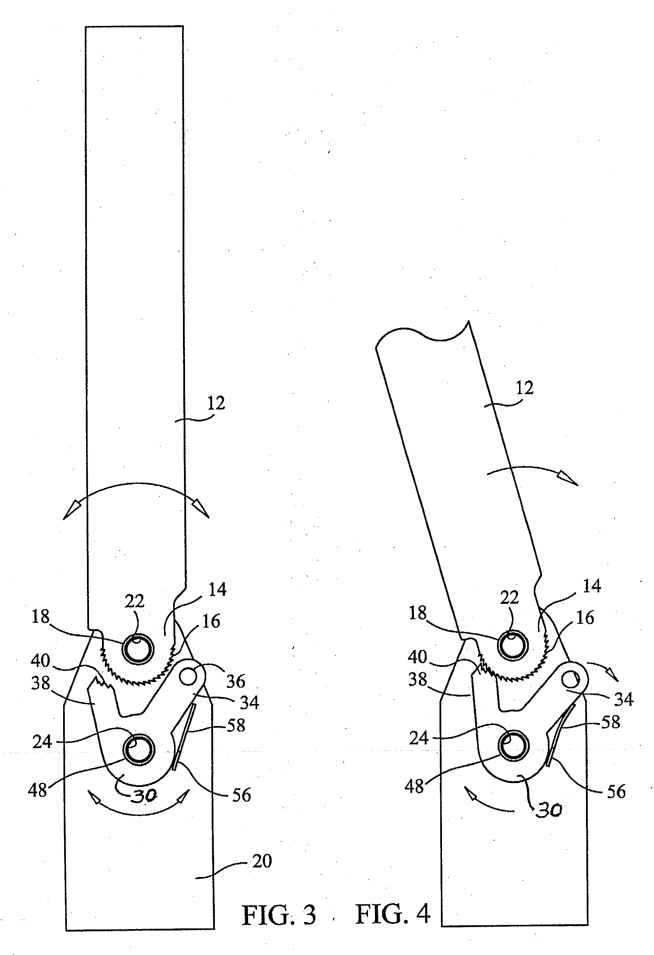

Figure 1 is an exploded view of an embodiment of the invention; and Figure 2 is a top view of various ones of the parts shown in Figure 1. The pressure activated ratchet mechanism illustrated in Figures 1 and 2,and the operation of which is shown in Figures 3 through 5, includes a pair of parts arranged for relative rotational movement with respect to one another. One of these parts is shown as an elongated lever 12; and the other is shown as a base member 20. These parts, while illustrated in a generally diagrammatic form in the various figures of the drawings, may be incorporated into a variety of different mechanisms. For example, the lever 12 could be a portion of the seat back of a lounge chair or pool chair, while the base member 20 could be an extension of the frame on which the seat back of such a chair is located. Alternatively, the lever 12 could be a portion of a ratcheting joint in a cervical brace, and the base member 20 another portion of the ratcheting joint of such a brace. The operation, however, is the same, whether the parts 12 and 20 are separate parts of a rotatable ratchet joint for attachment to some other component or are integral parts of such a component.

As illustrated in the various figures 1 through 5 of the drawing, the right-hand end (as viewed in Figure 1) of the lever 12 terminates in a portion 14 having a circular end with ratchet teeth 16 formed about the circumference. The ratchet teeth 16 face generally toward the right (as viewed in Figures 1 and 2) in the manner of conventional ratchet mechanisms arranged in a circular configuration. A hole 18 is formed concentrically with the circular end on which the ratchet teeth 16 are mounted; and this hole is designed to slip over a pivot post 22, which extends upwardly from the base member 20, as shown in Figure 1.

A pawl member 30 in the general configuration of an L-shaped lever or a V-shaped lever, has a hole 32 through it at the bight of two arms 34 and 38 which form the pawl member 30; and the hole 32 is dimensioned to fit pivotally over an upwardly extending pivot post 24 on the base 20. As is readily apparent from an examination of all of Figures 1,2,3 and 4, the pivot posts 22 and 24 are spaced apart from one another to permit engagement and disengagement of the pawl 30 and the ratchet teeth 16 by relative rotation of the pawl lever 30 with respect to the lower end 14 of the lever 12.

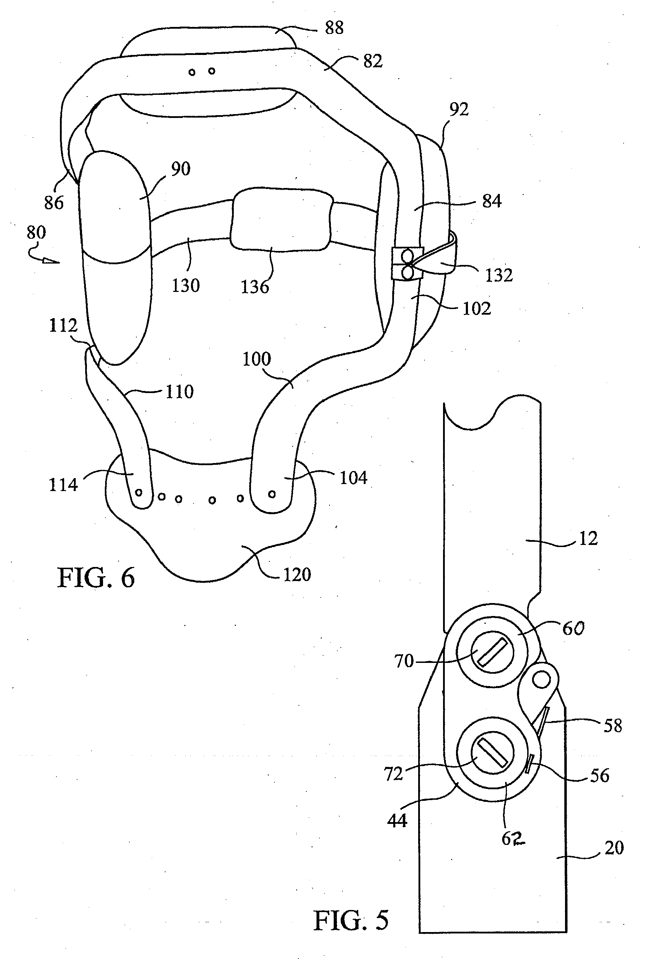

In the assembly of the pressure activated ratchet mechanism shown in Figure 1, the hole 18 through the lever arm 12 is slipped over the pivot post 22; and the hole 32 through the pawl lever 30 is slipped over the pivot post 24. A retainer 44, having corresponding holes 46 and 48 in it, then is placed over the top of the end 14 and over the pawl lever 30 to hold them in place. A leaf spring 54, in the form of a generally elongated rectangle 58, with an upward extension 56, is mounted with the extension 56 extending through a corresponding slot 50 in the retainer 44. Since the slot 50 is of rectangular configuration, the extension 56 is captivated in the position shown in Figures 3 and 4, which is the rest position of the spring 54. After the spring 54 is in place and the retainer 44 has been placed over the pivot posts 22 and 24, a pair of washers 60 and 62 are placed in recesses in the top of the retainer 44; and threaded fasteners, shown in the form of screws 70 and 72, are threaded into internally threaded openings in the pivot posts 22 and 24 to secure the assembly together, as shown in Figure 5.

In the relaxed position of the spring 56/58, the upper or rightward end (as shown in Figure 3) of the spring 58 rests against the arm 34 of the pawl 30 to bias the arm 38, which has pawl teeth 40 on its extremity, out of engagement with the teeth 16 on the end 14 of the ratchet lever 12. This allows the ratchet lever 12 to rotate freely in either direction, as indicated by the arrow across the lever 12, as shown in Figure 3. This is considered the release or rest position of the pawl and ratchet mechanism.

As shown in Figure 4,when the lever arm 34 of the rotatable pawl 30 is pulled by a suitable force or pressure (source of which is not shown), to the right and slightly downwardly in a clockwise direction, the pawl 30 rotates clockwise about the pivot post 24 to cause the pawl teeth 40 to engage the ratchet teeth 16 on the end 14 of the ratchet lever 12. As is apparent from an examination of Figure 2 and 3 particularly, the direction or orientation of the teeth 16 and 40 is such that the ratchet lever 12 is permitted to rotate in the direction of the arrow shown in Figure 4 (clockwise, as shown in Figure 4), but is prevented from opposite (counterclockwise) rotation because of the manner in which the teeth 40 on the lever arm 38 and the teeth 16 on the end 14 of the ratchet are oriented. As the force or pressure applied to the end of the arm 34 is increased to increase the clockwise rotational force (as shown in Figure 4) of the teeth 40 on the pawl 30 into engagement with the teeth 16 on the end of the ratchet lever 12, the holding force preventing rotation of the arm 12 in a reverse or counterclockwise direction, is increased. As soon as the pulling force or pressure indicated by the arrow adjacent the end of the lever arm 34, however, is released, the mechanism reverts back to the configuration shown in Figure 3 under the force of the spring 54. This allows full free rotation in either direction of the lever 12 relative to the base member 20.

It should be noted that while a leaf spring 54, comprising the elements 56 and 58, is indicated for applying the return or disengaging force to the pawl 30, other types of springs, such as coil springs (operated either in compression or extension), or a spiral spring around the pivot 24 may be used equally as well, and effectively as the leaf spring 54 which is illustrated in the drawings. Also, for some applications, the return force to the relative positions shown in Figure 3 can be applied magnetically or by gravity. The principle of operation is the same. The spring force which is applied to return the parts of the mechanism to the relative positions shown in Figure 3 is the force which initially needs to be overcome by application of a pulling force or pressure on the end of the lever arm 34, as shown in Figure 4. Once that force, however, is overcome, additional pressure applied to tend to rotate the pawl 30 in a clockwise direction (as shown in Figures 3 through 5) serves to more tightly engage the teeth 40 with the teeth 16 to prevent reverse relative rotation of the parts 12 and 20, as described previously.

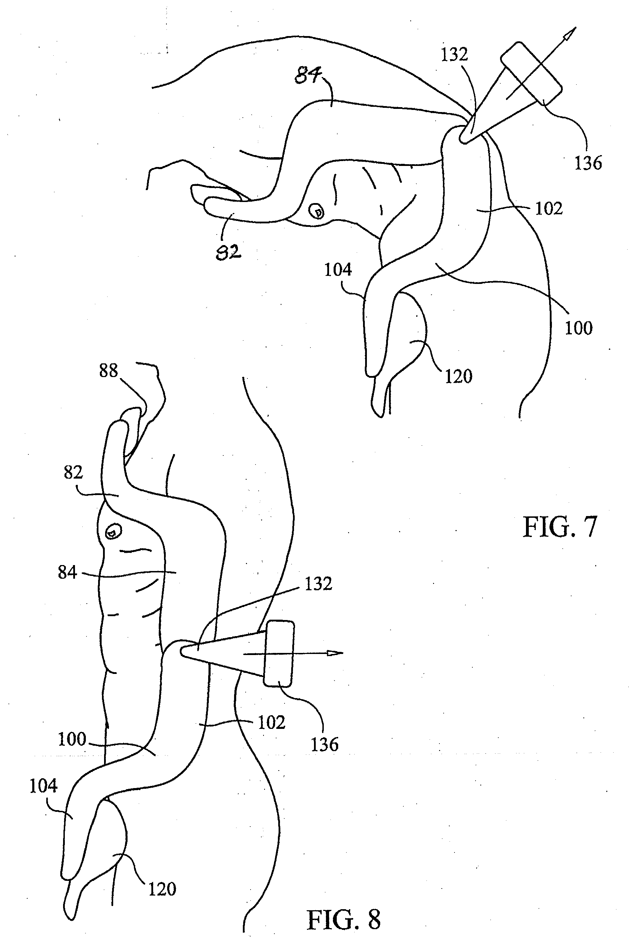

Figure 6 illustrates a typical configuration of a cervical brace comprising upper and lower parts, which are hinged together substantially at the mid point. The upper part comprises a generally U-shaped rigid member 82 having a front portion with a pad 88 on its reverse side, designed to rest against the upper chest of a user, as illustrated most clearly in Figure 8. The arms 82 then extend along the side of the wearer, and terminate in downwardly extending portions 84 and 86. Pads 90 and 92 typically are located on the inside of the brace to prevent abrasions, and to increase comfort. The lower terminus of the arms 84 and 86 is hingedly connected through a pressure activated ratchet mechanism of the type shown in Figures 1 through 5 to the upper ends of a pair of arms 102 and 112, each of which are formed as extensions of lower members 100 and 110. The lower ends of the members 100 and 110 extend forwardly and downwardly to terminate in arm portions 104 and 114 attached to a lower rigid pressure member 120, which also carries a pad on the side facing the wearer.

The back side of the cervical brace has a flexible belt or strap 130 on it, with opposite ends 132 attached to the lever arm 34 of the pressure activated ratchet mechanism shown in Figures 1 through 5. This attachment is effected in any suitable manner, including, but not limited to, a pin (not shown) extending from the end 132 through the hole 36 in the arm 34 of the pawl 30. The strap 130 carries a pad 136 approximately at its center point for resting against the back of the wearer, again, to provide comfort.

Typically, the wearer of the device rotates the parts 82 and 100 in a forward position, as shown in Figure 7, in order to apply the device. Since this is a cervical brace, the wearer generally is bent over in a forward position; and the brace is intended to assist the wearer in maintaining his or her back in a generally upright position. When the device is in the relative position shown in Figure 7, the pawl and ratchet mechanisms which are included at the pivots on the ends 132 of the strap 130 are in the released configuration shown in Figure 3. Thus, it is relatively easy for the wearer to place the device about the lower abdomen, as indicated in Figure 7, with the upper portion 93 in a forward direction.

The strap 130 across the back then is fastened together to apply pressure to the arm 34 of the pawl 30. This pulls the plate 120 against the pelvic region of the wearer; and the pressure force applied through the end 132 to the lever 34, pivots the pawl 30 in the direction shown in Figure 4 to engage the teeth 40 and 16 of the pawl and ratchet mechanism, as shown in Figure 4. This allows rotation of the member 82 in its clockwise direction (as shown in Figure 7 and as illustrated in Figure 4). It should be noted that the lever 12 of Figure 4 may be either incorporated integrally into the lower end 84 of the upper portion of the cervical brace; or it may be separately attached to the upper portion 84 of the cervical brace. Similarly, the base member 20, which is illustrated in Figures 1 through 5, may be an integral part of the arms 102 and 112 of the cervical brace; or a base member 20 of the type shown in Figures 1 through 5, alternatively may be separately attached to the arms 102 and 112. Irrespective of whether the ratchet mechanism is integrally formed as a part of the brace or is separately constructed and subsequently attached to the brace, the operation is the same.

Once the ends 132 are secured to the lever arms 34 of the pawl mechanism 30 on each side of the brace 80, rotation either in a step-by-step fashion, or directly to a pre-established position, with the assistance of a therapist or under control of the wearer himself or herself, allows the brace to apply pressure on the upper chest and the pelvic plate 120 to assist the wearer in attaining an upright position, as shown in Figure 8. The amount of rotation depends upon the nature of the therapy and support which is desired. In any event, reverse rotation (in the counterclockwise direction, as shown in Figure 7) is prevented so long as pressure is applied to hold the teeth 40 of the pawl 30 into engagement with the teeth 16 on the ratchet member 12. It also should be noted that, initially, a relatively lower amount of pressure is applied when the device is first being moved from the position shown in Figure 7 to the position shown in Figure 8. Any attempt, however, whether voluntarily or involuntarily, made by the wearer to bend forward meets with greater resistance, at increased pressure, to hold the teeth 40 and 16 into ever tighter engagement as the pressure is increased. Thus, the safety factor of the pawl mechanism increases as pressure increases.

Whenever removal of the cervical brace of Figures 6,7 and 8 is desired, all that is necessary is to release the strap 130 (by any suitable means provided to do this); and immediately the spring 56/58 returns the pawl 30 to the position shown in Figure 3 by rotating it in an counterclockwise direction (as viewed in Figures 3 and 4). This allows the wearer to bend forward or just simply drop the support on the upper portion of the chest to the position shown in Figure 7 for removal of the cervical brace.

Although the embodiment shown in Figures 6 to 8 specifically has been illustrated in conjunction with a cervical brace, the mechanism employed is suited for use in all of the various pawl and ratchet situations which previously have been described, and others which particularly will benefit from a pressure activated pawl mechanism. As mentioned above, the pressure activated mechanism increases in its safety (against reverse rotation or movement) as the pressure on the arm 34 is increased. It also should be noted that the principles which have been disclosed in conjunction with the embodiment shown may be applied to pressure activated linear pawl and ratchet operations as well. A rotary configuration is particularly suitable for pivoted joints; but situations exist which utilize a linear pawl and ratchet for lifting items from one position to another. The principles of operation are the same.

It also should be noted that while pivot posts 22 and 24 are shown affixed to the base member 20 provide the pivoting operation, a comparable operation also may be achieved by providing holes or recesses in the locations of one or both of the posts 22 and 24 on the member 20, with affixed mating pivot posts or pins at the locations of the holes 18 and 32 on the ratchet and the pawl 30, respectively, to achieve the same operating results.

The various parts also may be made of different materials, depending upon the particular application which is to be made of the pawl and ratchet mechanism. In many cases, the parts will be made of steel or aluminum,while for some applications, plastic or other materials capable of applying the desired amount of force for the particular application may be employed.

Various other changes and modifications will occur to those skilled in the art for performing substantially the same function, in substantially the same way, to achieve substantially the same result without departing from the true scope of the invention as defined in the appended claims.

## Claims

1. A ratchet mechanism **characterized by** a ratchet member (12) mounted for movement in two directions, a pawl member (30) mounted adjacent the ratchet member (12) out of engagement with the ratchet member (12) and responsive to predetermined pressure (via 34) to engage the ratchet member (12).

2. A ratchet mechanism according to Claim 1 further **characterized in that** the ratchet member (12) is mounted for rotational movement (on pivot 22).

3. The ratchet mechanism according to Claims 1 or2 further **characterized in that** the ratchet member (12) is mounted for limited rotational movement (on pivot 22).

4. The ratchet mechanism according to Claims 1,2 or 3 further **characterized by** a spring (58) normally biasing the pawl member (30) out of engagement with the ratchet member (12).

5. A ratchet mechanism according to any of the preceding claims further **characterized by** a common base (20) where the ratchet member (12) and the pawl member (30) are pivotally mounted (on pivots 22 and 24) adjacent one another on the base (20).

6. A ratchet mechanism according to any of the preceding claims further **characterized in that** the pawl member (30) is in the form of a generally L-shaped lever pivotally mounted (on 24) at the bight of the L, wherein one end (38) of the pawl member (30) is designed for engagement with the ratchet member (12), and the other end (34) of the pawl member is adapted to respond to the application of pressure thereto to rotate the pawl member (30) into engagement with the ratchet member (12).

## Amended claims

### Amended claims in accordance with Rule 86(2) EPC.

**1.** A ratchet mechanism comprising a common base (20), a ratchet member (12) mounted for movement in two directions, a pawl member (30) and a spring (58); the ratchet member (12) and the pawl member (30) being pivotally mounted on pivots (22, 24) adjacent one another on the base (20) and the spring (58) being mounted on the base (20) **characterized in that** the spring (58) normally biases the pawl member (30) out of engagement with the ratchet member (12), and the pawl member (30) is responsive to predetermined pressure overcoming the bias of the spring (58) to engage the ratchet member (12) with a tighter engagement as the pressure is increased.

**2.** A ratchet mechanism according to Claim 1 further **characterized in that** the ratchet member (12) is mounted for rotational movement (on pivot 22).

**3.** The ratchet mechanism according to Claims 1 or2 further **characterized in that** the ratchet member (12) is mounted for limited rotational movement (on pivot 22).

**4.** A ratchet mechanism according to any of the preceding claims further **characterized in that** the pawl member (30) is in the form of a generally L-shaped lever pivotally mounted on pivot (24) at the bight of the L, wherein one end (38) of the pawl member (30) is designed for engagement with the ratchet member (12), and the other end (34) of the pawl member is adapted to respond to the application of pressure thereto to rotate the pawl member (30) into engagement with the ratchet member (12).
